Europäisches Patentamt

(19) European Patent Office

Office européen des brevets

(11) Numéro de publication : **0 194 202 B1**

# (12) FASCICULE DE BREVET EUROPEEN

(45) Date de publication du fascicule du brevet :
06.02.91 Bulletin 91/06

(51) Int. Cl.$^5$ : **A61K 35/78**, // (A61K35/78, 33:08)

(21) Numéro de dépôt : 86400446.0

(22) Date de dépôt : 04.03.86

(54) **Composition pharmaceutique à base de gomme de guar et d'autres anti-acides pour la protection de la muqueuse oeso-gastro-duodénale.**

(30) Priorité : 06.03.85 FR 8503306

(43) Date de publication de la demande :
10.09.86 Bulletin 86/37

(45) Mention de la délivrance du brevet :
06.02.91 Bulletin 91/06

(84) Etats contractants désignés :
AT BE CH DE GB IT LI LU NL SE

(56) Documents cités :
FR-A- 2 073 254
FR-M- 7 794
H.D. FEIN: "Modern drug encyclopedia and therapeutic index", 1961, éd. 8, page 1169, The Reuben H. Donnelley Corp., New York, US.

(73) Titulaire : **Grimberg, Georges Serge**
**123 rue de l'Université**
**F-75007 Paris (FR)**

(72) Inventeur : **Grimberg, Georges Serge**
**123 rue de l'Université**
**F-75007 Paris (FR)**

(74) Mandataire : **Madeuf, Claude Alexandre Jean et al**
**CABINET MADEUF 3, avenue Bugeaud**
**F-75116 Paris (FR)**

## Description

La présente invention a pour objet une composition pharmaceutique à base de siméthicone pour la protection de la muqueuse oeso-gastro-duodénale.

Dans leur présentation actuelle, les compositions protectrices de la muqueuse oeso-gastro-duodénale se présentent sous des formes différentes, par exemple en suspension, en granulés, en comprimés, en poudre. Toutefois malgré ces différentes présentations on n'obtient pas le résultat recherché car la phase purement physique n'est pas toujours obtenue et la dispersion n'est pas bonne, ce qui ne permet pas de bien tapisser les muqueuses.

De plus, les différents anti-acides utilisés et leurs associations ne couvrent pas valablement les différentes zones de pH susceptibles de se produire chez un malade.

Conformément à l'invention, il est créé une composition comprenant par dose thérapeutique de la gomme de guar, de l'hydroxyde de magnésium, de l'hydroxyde d'aluminium et du phosphate d'aluminium. On utilise également de la gomme de guar comme anti-acide pour la zone de ph1 à ph0,8, mais différents autres agents anti-acides peuvent y être ajoutés.

Diverses autres caractéristiques de l'invention ressortent d'ailleurs de la description détaillée qui suit.

On donne ci-après plusieurs exemples de la composition pharmaceutique nouvelle à partir de la gomme de guar, de l'hydroxyde d'alumine, de l'hydroxyde de Mg et d'un ou plusieurs composants actifs pouvant également renfermer des gélifiants, des édulcorants et des aromatisants.

### Composition pharmaceutique ingérable :

| | | |
|---|---|---|
| - Hydroxyde d'alumine | 1.000 | g |
| - Gomme de guar en poudre | 2.478 | g |
| - Huile de silicone | 12,5 | g |
| - Mono-oléate de sorbitane | 6,44 | g |
| - Polysorbate 80 ® | 3,22 | g |
| - Silice micronisée | 50 | g |
| - Sucre | 6.000 | g |
| - Saccharinate de sodium | 10 | g |
| - Cyclamate de sodium | 40 | g |
| - Noix de coco (arôme en poudre) | 200 | g |

Lorsque le patient mélange le produit ainsi obtenu dans une phase aqueuse, la gomme de guar se disperse puis se met à gonfler et au moment du gonflement emprisonne l'hydroxyde d'alumine. Ce phénomène se poursuit in vivo.

On remarque que, dans cet exemple, la gomme de guar peut être diminuée en quantité et additionnée notamment d'alginates, de gélatine, de gommes ou de celluloses.

Mais il est remarquable de constater que le produit obtenu est dans tous les cas de belle apparence, fabriqué rapidement et sans irrégularités. Il peut être aisément conditionné et conservé en récipients, en vue de la dispersion lors de l'utilisation, ou transformé sous toute forme galénique convenable telle que comprimés, sachets.

Dans tous les cas, si on disperse la présente composition en milieu liquide, de l'eau par exemple, quelles que soient les proportions entre le produit et l'eau, la répartition sera obtenue rapidement à froid, de façon homogène, sans appareillage spécial. La consistance du gel obtenu variera pour un produit donné, d'une part, avec le temps, d'autre part, avec la quantité relative de poudre et d'eau.

Il suffit pour cela d'effectuer le mélange progressivement et de remuer ensuite ce mélange à l'aide d'une cuiller ou de la mesurette qui a servi au dosage, jusqu'à un premier degré d'épaississement du gel.

EXEMPLE 2

Une composition pharmaceutique est pratiquement identique si on remplace les 1.000 g d'hydroxyde

2

d'alumine par 500 g d'hydroxyde d'alumine plus 500 g d'hydroxyde de magnésium.

EXEMPLE 3

L'activité anti-acide de la gomme de guar confère au produit une activité très intéressante. En effet, les exemples cités ci-dessus montrent une zone d'activité antiacide très étendue.

La formule suivante, par dose thérapeutique permet de dégager quatre zones d'activité anti-acides :
a) hydroxyde de magnésium 0,5 g
b) hydroxyde d'aluminium 0,5 g
c) phosphate d'aluminium 0,3 g
d) gomme de guar (enrobée ou non enrobée) 0,2 g
et trois zones de pouvoir tampon :
a) de pH 3,5 à pH 3
b) de pH 2 à pH 1,6
c) de pH 1 à ph 0,8
La technique de mesure de pH est celle de Vattier.

Cette composition présente d'autres avantages et, notamment, une cinétique de libération d'activité lente de 30 minutes et, même après ce délai, il reste 36 à 20% d'activité selon le pH d'intervention.

On peut associer à ces quatre anti-acides différentes substances telles que le siméthicone qui possède une excellente activité sur le météorisme et les gaz.

On peut associer à la gomme de guar différents anti-acides.

Les essais réalisés sur animaux, avec la formule décrite, ont donné d'excellents résultats et ont montré que, par exemple, chez le rat ainsi que chez le chien auxquels on avait fait ingérer des agents provoquant une rapide ulcération de la muqueuse gastrique, le traitement pendant deux ou trois semaines avec la composition conforme à l'invention avait permis un rétablissement complet ou tout au moins une diminution très marquée des ulcérations.

Actuellement, et bien que les essais cliniques chez l'homme ne soient pas terminés, la composition conforme à l'invention permet, dans plus de 90% des cas cliniques observés, de définir avec exactitude une amélioration rapide et parfois définitive des parties atteintes de la muqueuse oeso-gastro-duodénale.

A ce sujet, la composition de l'exemple 3 s'est montrée parfaitement efficace sur trois sujets auxquels on avait injecté de la pentagastrine.

Ainsi, la composition 3 conforme à l'invention a permis de traiter deux malades caractérisés par des hypersécrétions de la muqueuse gastrique correspondant à une hyperacidité importante. La posologie a été de trois comprimés par jour pendant quinze jours.

## Revendications

1. Composition pharmaceutique à base de siméthicone, caractérisée en ce qu'elle comprend les ingrédients suivants par dose thérapeutique :

| | |
|---|---|
| – gomme de guar | 0,2 g |
| – hydroxyde de magnésium | 0,5 g |
| – hydroxyde d'aluminium | 0,5 g |
| – phosphate d'aluminium | 0,3 g |

2. Composition pharmaceutique selon la revendication 1, caractérisée en ce qu'elle renferme différents excipients qui lui donnent une forme galénique pharmaceutique acceptable par le malade.

3. Composition pharmaceutique selon la revendication 1, caractérisée en ce que la gomme de guar est utilisée comme anti-acide pour la zone de ph 1 à ph 0,8 avec possibilité d'ajouter différents autres agents anti-acides.

4. Composition pharmaceutique selon l'une des revendications 1 à 2, caractérisée en ce qu'elle est appliquée en tant qu'antiacide protecteur de la muqueuse oeso-gastro-duodénale et contre le météorisme.

## Ansprüche

1. Pharmazeutische Zusammensetzung auf der Basis von Simethikon, dadurch gekennzeichnet, dass sie folgende Bestandteile pro therapeutischer Dosis aufweist :

```
- Guargummi          0,2 g
- Magnesiumhydroxid  0,5 g
- Aluminiumhydroxid  0,5 g und
- Aluminiumphosphat  0,3 g.
```

2. Pharmazeutische Zusammensetzung nach Anspruch 1, dadurch gekennzeichnet, dass die verschiedene lösende Mittel aufweist, die ihr eine galenische und pharmazeutische Form geben, die für den Patienten akzeptabel ist.

3. Pharmazeutische Zusammensetzung nach Anspruch 1, dadurch gekennzeichnet, dass Guargummi als Antiazidum im Bereich von ph 1 bis ph 0,8 verwendet wird, dem verschiedene andere antiazidische Wirksubstanzen zugegeben werden können.

4. Pharmazeutische Zusammensetzung nach einem der Ansprüche 1 bis 2, dadurch gekennzeichnet, dass sie angewendet wird als antiazidischer Schutz der Schleimhaut im Speiseröhren-, Magen-Darm- und Zwölffingerdarmbereich und gegen Meteorismus.

## Claims

1. Simethicone base pharmaceutical composition characterized in that it comprises the following components for a therapeutical dose :

```
- guar gum            0.2 g
- magnesium hydroxide  0.5 g
- aluminium hydroxide  0.5 g
- aluminium phosphate  0.3 g
```

2. Pharmaceutical composition according to claim 1, characterized in that it contains various excipients giving to it a galenic form pharmaceutically acceptable by the patient.

3. Pharmaceutical composition according to claim 1, characterized in that gum guar is used as an antacid for the pH 1 to pH 0.8 zone with possibility to add various other antacid agents.

4. Pharmaceutical composition according to one of claims 1 to 2, characterized in that it is applied as an antacid for protecting the oeso-gastro-duodenal-mucous membrane and against meteorism.